# EUROPEAN PATENT APPLICATION

(11) **EP 4 509 606 A1**
(43) Date of publication of application: **19.02.2025**
(21) Application number: 23791124.3
(22) Date of filing: 13.04.2023
(51) Int. Cl.: C12N 15/57, C12N 15/52, C07K 14/745, A61K 48/00

(54) **NUCLEIC ACID CONSTRUCT FOR TREATING HEREDITARY COAGULATION FACTOR DEFICIENCY AND USE THEREOF**

(30) Priority: 19.04.2022 WO PCT/CN2022/087764; 19.04.2022 WO PCT/CN2022/087767
(71) Applicant: Kanglin Biotech (Hangzhou) Co., Ltd., Hangzhou, Zhejiang 310018 (CN)
(72) Inventor: WU, Haoquan, Hangzhou, Zhejiang 310018 (CN); SU, Lingling, Hangzhou, Zhejiang 310018 (CN); DING, Yanfu, Hangzhou, Zhejiang 310018 (CN); FU, Xiaobin, Hangzhou, Zhejiang 310018 (CN)
(74) Representative: Bayramoglu et al.
(86) International application number: PCT/CN2023/088206
(87) International publication number: WO 2023/202469

(57) **Abstract**

The present invention relates to the technical field of gene therapy, and in particular, to a nucleic acid construct for treating hereditary coagulation factor deficiency and a novel use thereof. The construct comprises the following elements: a polynucleotide encoding coagulation factor IX; and an AAV cis-regulatory element comprising one or more of an ITR, a promoter, a kozak sequence, an SV40 poly A, a UTR, or a WPRE. Preferably, the sequence of the polynucleotide encoding coagulation factor IX is a sequence that has undergone codon optimization. Preferably, the polynucleotide sequence that has undergone codon optimization is a sequence of coagulation factor IX comprising an R338L mutation. The novel use is use of an AAV vector serotype targeting muscle in preparing a hereditary coagulation factor deficiency therapeutic product. An adeno-associated virus prepared from the nucleic acid construct and a nucleic acid construct carrying a coagulation factor target gene may have a muscle targeting property in an intramuscular injection delivery mode, and help express the coagulation factor at a therapeutically effective amount.

## Description

### Technical Field

The present invention relates to the technical field of gene therapy, in particular to a nucleic acid construct for treating hereditary coagulation factor deficiency and use thereof.

### Background Art

Platelet coagulation factors are various protein components involved in the blood coagulation process. These factors work together to maintain normal coagulation physiology in a hemorrhagic state. The deficiency of one or more coagulation factors may lead to a group of inherited hemorrhagic diseases, also referred to as hemophilia. Hemophilia A, which is the most common in clinical practice, is an X-linked recessive genetic disease caused by the deficiency of a coagulation factor VIII. Patients have coagulopathy of varying severity and spontaneous bleeding. Hemophilia B is an X-linked recessive hereditary hemorrhagic disease caused by the deficiency or quality defect of the FIX amount in plasma resulting from a gene mutation of a coagulation factor IX, with an incidence rate of about 1/30,000 in men.

Coagulation factor replacement therapy, as the only treatment means for hemophilia at present, includes both an early plasma-derived (pd) coagulation factor and a recombinant human coagulation factor protein product produced by genetic engineering. These products are expensive, and may produce inhibiting factors when used over time. Gene therapy using AAV or lentivirus vectors as means of delivery may take an effect of continuously expressing fully functional coagulation factors by permanent integration of genes coding coagulation factor proteins into human cells or long-term implantation into non-dividing cells. Due to its predictable long-term effect and safety, the gene therapy has become the most promising treatment means for hemophilia.

In recent years, in the clinical application of hemophilia, AAV vector delivery with the liver as a target organ has gradually become the mainstream trend, and BioMarin's BMN 270, UniQure's AMT-061 and other star products have been carried out to phase III clinical trials. However, this technical route cannot avoid the risk of hepatotoxicity from a drug. On November 16, 2020, Nature Biotechnology, a top international academic journal, published a research paper entitled: A long-term study of AAV gene therapy in dogs with hemophilia A identifies clonal expansions of transduced liver cells. In this study, an AAV virus gene therapy test was conducted in dogs with hemophilia A. However, during the 10-year post-treatment observation, the research team found that some of the therapeutic gene fragments carried by an AAV virus were integrated into dog's chromosomes in the vicinity of genes that control the growth, resulting in the potential to induce cancers. In addition, at present, the main treatment method of hemophilia B is also coagulation factor replacement therapy, in which coagulation factors have undergone a development course of blood source → recombination → long acting, but patients need lifelong medication. Although this method can control a disease to a certain extent, there is still a risk of bleeding, thereby also bringing many side effects and a great economic burden to patients. Therefore, many gene therapy companies around the world are trying to deliver functional copies of a FIX gene to the bodies of patients in a one-time manner by using virus vectors, so that the liver can express missing coagulation factors, thereby eliminating the risk of bleeding of patients and achieve therapeutic benefits. At present, several gene therapy drugs (e.g., Pfizer (Spark)'s SPK-9001-LTFU101 and Uniqure's AMT-061) in clinical practice have also progressed to the clinical stage. In particular, Uniqure's AMT-061 has entered phase III clinical trials. On December 22, 2020, uniQure announced the suspension of its phase III clinical trial for the treatment of hemophilia B, because a case of liver cancer patient was reported in a clinical trial of AMT-061.

In order to avoid liver toxicity and hidden dangers of liver cancers, hemophilia gene therapy must explore safer and more effective AAV vectors and administration methods, and change the tissue distribution of AAV drugs in the human body, so as to make it become a gene therapy drug with significant safety advantages. In addition, the amount of FIX expressed by the existing AAV after delivery to the body is relatively small, and cannot meet the plasma concentration required for the treatment of a disease, so it is necessary to develop AAV that can meet the plasma concentration required for the treatment of a disease.

### Content of the Invention

In view of the shortcomings of the prior art described above, an object of the present invention is to provide a nucleic acid construct for treating hereditary coagulation factor deficiency, and new use of an AAV carrier serotype targeting muscles, which are used to solve the problems in the prior art.

In order to achieve the above and other related purposes, the present invention provides a nucleic acid construct comprising the following elements:
a polynucleotide coding a coagulation factor IX; and
an AAV cis-regulatory element, comprising one or more of an ITR, a promoter, a kozak sequence, an SV40 polyA, a UTR, or a WPRE.

Preferably, a sequence of the polynucleotide coding the coagulation factor IX is a sequence that has undergone codon optimization; preferably, the sequence of the polynucleotide that has undergone codon optimization is a sequence of the coagulation factor IX comprising an R338L mutation; and preferably, the sequence of the polynucleotide that has undergone condon optimization is shown in SEQ ID NO: 23 or a sequence with more than 95% identity to the sequence shown in SEQ ID NO: 23.

The promoter is selected from a mammalian constitutive promoter, a mammalian tissue-specific promoter or a mammalian inducible promoter.

A structure of the nucleic acid construct is selected from any of the followings:
1) ITR-promoter-kozak sequence-F9IDTM gene expression cassette-SV40 polyA sequence-ITR;
2) ITR-promoter-kozak sequence-F9IDTM gene expression cassette-WPRE-SV40 polyA sequence-ITR; and
3) ITR-promoter-UTR sequence-kozak sequence-F9IDTM gene expression cassette-WPRE-SV40 polyA sequence-ITR.

The present invention provides use of an AAV vector serotype targeting muscles in the preparation of a hereditary coagulation factor deficiency therapeutic product.

The AAV vector serotype targeting muscles is selected from AAV1, AAV2, AAV6, AAV6.2FF, AAV7, AAVrh74, AAV8, AAV9 or mutants thereof.

The hereditary coagulation factor deficiency therapeutic product is selected from a nucleic acid construct or an adeno-associated virus.

The hereditary coagulation factor deficiency is selected from hemophilia A, hemophilia B or hemophilia C.

The present invention further provides a nucleic acid construct for the preparation of a hereditary coagulation factor deficiency therapeutic product, wherein an adeno-associated virus prepared from the nucleic acid construct has a muscle targeting property and expresses a coagulation factor at a therapeutically effective amount; and preferably, the nucleic acid construct has a muscle targeting property under an intramuscular injection delivery mode.

The present invention further provides an adeno-associated virus vector expression system, comprising the two nucleic acid constructs as described above.

The present invention further provides an adeno-associated virus, which is packaged by the adeno-associated virus vector expression system.

The present invention further provides a cell line, which is a cell line infected by the adeno-associated virus.

The present invention further provides use of the adeno-associated virus and the cell line in the preparation of a product for the prevention and treatment of hereditary coagulation factor deficiency.

The present invention further provides a method for treating hereditary coagulation factor deficiency, comprising: administrating a patient with an effective amount of the adeno-associated virus.

As described above, the present invention provides new use of an AAV vector serotype targeting muscles, and has the following beneficial effects: safer and more effective AAV vectors and administration methods are provided to change the tissue distribution of an AAV drug in the human body, so as to make it become a gene therapy drug with significant safety advantages. An expression quantity of FIX is increased to a large enough amount, thereby meeting the plasma concentration required to treat a disease. The usage amount of AAV is reduced while achieving a therapeutic effect. No hepatotoxicity will be caused, so the present invention is safer and more effective than existing treatment methods.

### Description of the Drawings

FIG. 1 shows a schematic diagram of the design of an adeno-associated virus vector of a coagulation factor IX gene in the present invention.
FIG. 2 shows a pAAV-MCS-ITR-130-CAG-SV40 plasmid profile in the present invention.
FIG. 3 shows expression quantities of FIX of different constructs in a C2C12 cell supernatant in the present invention.
FIG. 4 shows a schematic diagram of the design of different promoters for the adeno-associated virus vector of the coagulation factor IX gene in the present invention.
FIG. 5 shows expression quantities of FIX of constructs of different promoters in the C2C12 cell supernatant.
FIG. 6 shows the effects of different expression vectors on FIX activity in mouse.
FIG. 7 shows a comparison of expression levels of different promoters in animals.
FIG. 8-1 to FIG. 8-2 show schematic diagrams of target gene constructs for verifying the bio-distribution of different serotypes and vector delivery efficiency in the present invention, wherein FIG. 8-1 is a schematic structural diagram of KL-pAAV-HA21 and FIG. 8-2 is a schematic structural diagram of KL-pAAV-Luc.
FIGS. 9-1 to 9-4 show in-vivo imaging patterns of Balb/c mouse at Day 3, Day 7, Day 14, and Day 21 after being injected with different AAVs respectively in the present invention.
FIG. 10-1 to FIG. 10-2 show the FIX activity and expression quantities of plasma concentrations of hemophilia B model F9-KO3 mouse injected with AAV within Week 1, Week 2, Week 4, Week 6, and Week 8.
FIG. 11 shows APTT assay results at Week 8 after administration.
FIG. 12 shows bleeding assay results in a tail-clip test at Week 4 after administration.
FIG. 13 shows TEG results in a thromboelastogram at Week 4 after administration, wherein R(min) represents the onset time of coagulation response in the blood of various experimental animals.
FIG. 14 shows DNA tissue distribution assay results of AAV at Week 1, Week 4, and Week 8 after administration.

### Specific Implementations

The present invention provides a nucleic acid construct, including the following elements: a polynucleotide coding a coagulation factor IX; and an AAV cis-regulatory element, wherein a sequence of the polynucleotide coding the coagulation factor IX is a sequence that has undergone codon optimization, and the AAV cis-regulatory element comprises an expression control element. In one embodiment, the expression control element may be a tissue-specific expression control element. The tissue-specific expression control element is, for example, a muscle tissue-specific expression control element. The tissue-specific expression control element comprises any one or more of an ITR, a promoter, a kozak sequence and an SV40 polyA, and the nucleic acid construct is a nucleic acid construct for preparing an adeno-associated virus for intramuscular injection administration.

In the present invention, the term "nucleic acid construct" refers to an artificially constructed nucleic acid segment that may be introduced into a target cell or tissue.

The coagulation factor is selected from a coagulation factor VIII, a coagulation factor IX or a coagulation factor XI and mutants thereof.

The coagulation factor IX (or referred to as FIX) comprises 433 amino acids and a signal peptide with a length of 28 amino acids.

In one embodiment, the coagulation factor IX is a coagulation factor IX mutated with R338L. The coagulation factor IX with the R338L mutation mutates the 338^{th} amino acid of FIX from arginine R to leucine L, and the coagulation factor IX after this mutation may be abbreviated as FIX-R338L. The titer of FIX on mouse can be increased by more than 5 times after the mutation at this site.

In one embodiment, a sequence of the polynucleotide (also referred to as F9IDTM) coding FIX-R338L that has undergone codon optimization is shown in SEQ ID NO: 23 or a sequence with more than 95% identity to the sequence shown in SEQ ID NO: 23.

In one embodiment, the promoter is selected from a mammalian constitutive promoter, a mammalian tissue-specific promoter, and a mammalian inducible promoter.

The mammalian constitutive promoter is selected from any of the followings: CMV (cytomegalovirus), EF1α (elongation factor-1α), EFS, CAG (consisting of a cytomegalovirus enhancer and a chicken β-actin promoter), CAGG, CBH, SFFV, MSCV, mPGK; and hPGK or UBC (ubiquitin C).

In a preferred embodiment, the mammalian tissue-specific promoter is selected from a muscle-specific promoter. Since the currently reported AAV targets the liver to cause hepatotoxicity and hidden dangers of liver cancers, it is necessary to seek other safe and effective administration methods. The inventors of the present application have studied a variety of administration methods, and finally concluded that AVV targeting muscle cells, which is administrated by intramuscular injection, is a good choice for the treatment of hemophilia B, so a muscle-specific promoter is selected accordingly in the construction of nucleic acid constructs.

The muscle-specific promoter is selected from tMCK, Desmin, SKCRM 4-DES, tMCK2, CK8, or MCK.

It is believed that in some examples, the ITR sequence may be folded into a hairpin structure which is a cis-regulatory element required for DNA replication initiation of AAV and packaging of recombinant AAV virus particles.

The ITR has a length of 130-145 bp. For example, the ITR has a length of 130-135 bp, 135-140 bp, 141 bp, 142 bp, 143 bp, 144 bp, or 145bp.

The lengths of the upstream ITR and the downstream ITR in the ITR may be the same or different.

In a preferred embodiment, the lengths of the upstream ITR and the downstream ITR are the same, and both are 141 bp.

In one embodiment, the AAV cis-regulatory element in the nucleic acid construct further includes UTR.

The UTR may introduce any UTR from any gene known in the art into the nucleic acid construct.

In one embodiment, the UTR is located after a promoter.

An untranslated region (UTR) is transcribed but not translated. Typically, 5' UTR starts at a transcription initiation site and terminates at an initiation codon, and 3' UTR starts immediately after a termination codon and continues until a transcription termination signal is obtained.

In some embodiments, the 5' UTR in a virus genome comprises a kozak sequence. In other embodiments, the 5' UTR in the virus genome does not contain a kozak sequence.

In one embodiment, the AAV cis-regulatory element in the nucleic acid construct further comprises WPRE. In one embodiment, the WPRE is located between a F9IDTM gene expression cassette and SV40 polyA.

A WPRE element can enhance the expression of a vector and the stability of transcribed mRNA, and improve the efficiency of mRNA splicing.

In one embodiment, a structure of the nucleic acid construct is selected from any of the followings:
1) ITR-promoter-kozak sequence-F9IDTM gene expression cassette-SV40 polyA sequence-ITR;
2) ITR-promoter-kozak sequence-F9IDTM gene expression cassette-WPRE-SV40 polyA sequence-ITR; and
3) ITR-promoter-UTR sequence-kozak sequence-F9IDTM gene expression cassette-WPRE-SV40 polyA sequence-ITR.

In some embodiments, the nucleic acid construct is an adeno-associated virus vector. In some embodiments, the adeno-associated virus vector also comprises a vector backbone.

In some embodiments, the vector backbone may be self-made or selected from suitable vector backbones sold in the market.

In one embodiment, the vector backbone is pAAV-MCS-ITR-130-CAG-SV40 and has a nucleotide sequence shown in SEQ ID NO: 1.

In one embodiment, the nucleotide sequence of the nucleic acid construct is selected from any one of the followings, as well as a nucleotide sequence having at least 75%, 80%, 85%, 90%, 95%, 96%, 97%, 98%, or 99% homology with any of the followings: SEQ ID NO: 22, SEQ ID NO: 3, SEQ ID NO: 5, SEQ ID NO: 8, SEQ ID NO: 15, SEQ ID NO: 17, SEQ ID NO: 19 and SEQ ID NO: 21.

The present invention further provides use of an AAV vector serotype targeting muscles in the preparation of a hereditary coagulation factor deficiency preventive or therapeutic product, or provides use of an AAV vector serotype targeting muscles in the prevention or treatment of hereditary coagulation factor deficiency.

The term "serotype" is a distinction used to refer to AVV having a capsid that is serologically distinct from other AVV serotypes. Serological distinction is based on the absence of cross-reactivity between antibodies to one AAV compared to another AVV. This cross-reactivity distinction is usually caused by differences in capsid protein sequences/antigenic determinants (e.g., by differences in VP1, VP2, and/or VP3 sequences of AAV serotypes). Although AAV variants comprising capsid variants may be serologically indistinguishable from reference AAV or other AAV serotypes, they differ by at least one nucleotide or amino acid residue from reference or other AAV serotypes.

Under the traditional definition, a serotype refers that a target virus has been tested for neutralizing activity in serum which is specific for all existing and characterized serotypes, and no antibodies that can neutralize the target virus have been found. Because more naturally occurring virus isolates are discovered and/or produced by capsid mutations, they may or may not be serologically different from any of the existing serotypes. Therefore, when there is no serological difference in a new virus (e.g., AAV), the new virus (e.g., AAV) may be a subtype or variant of the corresponding serotype. In many cases, a serological test for neutralizing activity needs to be performed on a mutant virus modified by a capsid sequence so as to determine whether it is another serotype in the traditional serotype definition. Correspondingly, for convenience and to avoid duplication, the term "serotype" broadly refers to serologically distinct viruses (e.g., AAV) and viruses (e.g., AAV) that are not serologically distinct and belong to subtypes or variants of a particular serotype.

In one embodiment, the AAV vector serotype targeting muscles is selected from AAV1, AAV2, AAV6, AAV6.2FF, AAV7, AAVrh74, AAV8, AAV9, or mutants thereof.

In a preferred embodiment, the AAV vector serotype targeting muscles is AAV6.2FF. AAV6.2FF is a recombinant adeno-associated virus triple mutation vector. By using a Firefly-Luciferase reporter vector packaged with this serotype capsid, it is found that the AAV6.2FF serotype vector has more muscle targeting properties and less liver targeting properties in wild-type c57 mouse using an in-vivo imaging technology.

The "targeting property" refers to the specificity of the AAV capsid protein present in AAV virus particles for the infection of a particular type of cells or tissues.

In the present invention, the "muscle targeting property" refers to the targeting property to muscle cells or tissues.

The muscle cells are selected from myocytes, myotubes, myoblasts or satellite cells.

The muscle tissues are selected from cardiac or skeletal muscle tissues. Muscle tissues account for 35%-40% of the body's weight, and have the potential to be used as a "biofactory" suitable for gene therapy. On the other hand, from the perspective of risk management, even if a hereditary coagulation factor deficiency therapeutic product causes a serious local adverse reaction, the cost of muscle tissue excision will be much lower than that of the liver, and the tumors in the muscle tissues are often benign and do not spread malignantly. In summary, the risk implications for the safety of targeted intramuscular administration are lower.

The hereditary coagulation factor deficiency therapeutic product is selected from nucleic acid constructs and adeno-associated viruses.

The hereditary coagulation factor deficiency is selected from hemophilia A, hemophilia B or hemophilia C.

The present invention further provides a nucleic acid construct for the preparation of a hereditary coagulation factor deficiency therapeutic product. An adeno-associated virus prepared from the nucleic acid construct has a muscle targeting property and expresses a coagulation factor at a therapeutically effective amount.

The nucleic acid construct has a muscle targeting property under an intramuscular injection delivery mode.

The therapeutically effective amount refers to an amount that is effective in the treatment of hereditary coagulation factor deficiency.

A dose used to achieve a therapeutic effect, e.g., a dose per kg body weight (vg/kg) of a vector genome, varies based on several different factors, including, but not limited to: an administration route, a heterologous polynucleotide expression level required to achieve the therapeutic effect, specific diseases being treated, any host immune response against virus vectors, host immune response to heterologous polynucleotides or expression products (proteins), and stability of expressed proteins. Those skilled in the art can determine a dose range of the rAAV/vector genome to treat patients with specific diseases or disorders based on these and other factors. In general cases, the dose range is at least 1×10⁸ or more, such as 1×10⁹, 1×10¹⁰, 1×10¹¹, 1×10¹², 1×10¹³, or 1×10¹⁴ or more vector genomes per kg body weight (vg/kg) to achieve therapeutic effects. An effective dose range of AAV in mouse is 1×10¹⁰-1×10¹¹, and 1×10¹²-1×10¹³ in dog.

Taking hemophilia B as an example, it is generally believed that in order to achieve a therapeutic effect, the concentration of a coagulation factor needs to be greater than 1% of the concentration of a normal individual factor so as to change a severe disease phenotype to a mild phenotype. Severe phenotype features are joint damage and fatal bleeding. In order to transition a mild disease phenotype to a slight phenotype, it is believed that the concentration of the coagulation factor needs to be greater than 5% of the normal concentration. For the treatment of these hemophilia subjects, the usual dose is at least 1×10¹⁰ vector genome (vg) per kg (vg/kg) host body weight, or about 1×10¹⁰ to 1×10¹¹ vg/kg host body weight, or about 1×10¹¹ to 1 ×10¹² vg/kg host body weight, or about 1×10¹² to 1×10¹³ vg/kg host body weight so as to achieve a desired therapeutic effect.

The dose of "effective" or "sufficient" amount (e.g., alleviating or providing therapeutic benefits or improvements) may generally respond effectively to one, more or all of adverse disease symptoms, consequences or complications to a measurable degree, one or more of adverse symptoms, disorders, diseases, pathologies or complications, such as those caused by or related to diseases, provided that the decrease, reduction, inhibition, suppression, restraining, limitation or control of disease progression or exacerbation is a satisfactory outcome.

The effective or sufficient amount can be provided in single administration (but is not required), may require a plurality of administrations and can be used alone or in combination with other components (e.g., therapeutic agents), therapeutants, protocols or therapeutic regimens, but this is not required. For example, the number may increase proportionally with a subject, type, status and severity of a disease being treated, or the need for side effects (if any) of treatment. In addition, if the effective or sufficient amount is administered in a single or multiple dose without a second composition (e.g., another drug or reagent), therapeutant, protocol or therapeutic regimen, it is unnecessary be effective or sufficient because it may include additional doses, quantities or durations over or beyond that dose, or may include additional components (e.g., drugs or reagents), therapeutants, protocols or therapeutic regimens so as to consider effective or sufficient amounts for a given subject. Amounts considered effective also include amounts that result in a reduction in other therapeutants, therapeutic regimens or protocols, such as administrating a recombinant coagulation factor protein (e.g., FVIII) for the treatment of coagulation disorders (e.g., hemophilia A).

In some embodiments, the nucleic acid construct is an adeno-associated virus vector. In some embodiments, the nucleic acid construct comprises a vector backbone expressing an adeno-associated virus capsid protein; and preferably, the adeno-associated virus capsid protein vector backbone expresses an adeno-associated virus capsid protein targeting muscle tissues.

In some embodiments, the vector backbone may be self-made or selected from suitable vector backbones sold in the market.

In some embodiments, the vector backbone is a vector backbone targeting muscles. The vector backbone is, for example, KL-pAAV-RC1, KL-pAAV-RC2, KL-pAAV-RC6, KL-pAAV-RC6.2FF, KL-pAAV-RC7, KL-pAAV-RCrh74, KL-pAAV-RC8, or KL-pAAV-RC9, having nucleotide sequences shown in SEQ ID NOs.1-8 in sequence.

The present invention further provides an adeno-associated virus vector expression system, comprising the nucleic acid construct for the preparation of a hereditary coagulation factor deficiency therapeutic product.

The adeno-associated virus vector expression system is selected from an Escherichia coli expression system, a yeast expression system, an insect expression system, a mammalian expression system and a plant expression system; and preferably, the adeno-associated virus vector expression system is selected from any one of a plasmid transient transfection expression system, a baculovirus expression system, a stable transfection cell line expression system, an adenovirus expression system, and an poxvirus expression system.

Further, the lentivirus vector system further includes a host cell. The host cell carries an adeno-associated virus vector. The host cell may be selected from various applicable host cells in the art, as long as the purpose of the present invention is not restricted. Specific applicable cells (e.g., 293T cells) may be cells that produce an adeno-associated virus.

In the plasmid transient transfection expression system, the adeno-associated virus vector expression system also comprises a nucleic acid construct carrying a target gene, and a helper plasmid. In an embodiment of the present invention, the nucleic acid construct carrying the target gene comprises a polynucleotide coding a coagulation factor and an AAV cis-regulatory element. Specifically, the nucleic acid construct carrying the target gene is a construct which comprises a polynucleotide coding a coagulation factor and an AAV cis-regulatory element described above in the present invention.

Further, the helper plasmid comprises an essential virus packaging component, and the helper plasmid may comprise a packaging plasmid.

The present invention further provides an adeno-associated virus (AAV). The adeno-associated virus is formed by packaging an adeno-associated virus vector expression system with a virus. The adeno-associated virus may be used to treat various diseases, such as hereditary coagulation factor deficiency.

The present invention further provides a cell line that is infected by an adeno-associated virus.

In one embodiment, the cell line is a mammalian cell line.

The present invention further provides use of the adeno-associated virus vector expression system, the adeno-associated virus and the cell line in the preparation of a product for the prevention and treatment of hereditary coagulation factor deficiency.

In one embodiment, the hereditary coagulation factor deficiency is hemophilia A, hemophilia B or hemophilia C. In one embodiment, the hereditary coagulation factor deficiency is mammalian hereditary coagulation factor deficiency. Furthermore, the hereditary coagulation factor deficiency is human hereditary coagulation factor deficiency.

The present invention further provides a method for treating hereditary coagulation factor deficiency, comprising: administrating a patient with the adeno-associated virus at an effective amount.

The hereditary coagulation factor deficiency is hemophilia A, hemophilia B or hemophilia C. In one embodiment, the hereditary coagulation factor deficiency is mammalian hereditary coagulation factor deficiency. Furthermore, the hereditary coagulation factor deficiency is human hereditary coagulation factor deficiency.

In one embodiment, the method comprises: injecting an effective amount of the adeno-associated virus into muscles of a patient.

The following specific examples are intended to illustrate the embodiments of the present invention. Those skilled in the art can easily understand the other advantages and effects of the present invention from the content disclosed in the present description. The present invention may also be implemented or applied by different specific embodiments, and various details in the present description may also be modified or changed based on different views and applications, without departing from the spirit of the present invention.

Before further describing the specific embodiments of the present invention, it should be understood that the protection scope of the present invention is not limited to the specific embodiments described below. It should also be understood that the terms used in the examples of the present invention are intended to describe a specific embodiment, but not to limit the protection scope of the present invention. In the description and claims of the present invention, unless otherwise expressly stated herein, the singular forms "a", "one" and "this" include the plural form.

When an example gives a range of values, it should be understood that, unless otherwise stated in the present invention, two endpoints of each range of values and any value between the two endpoints may be selected. Unless otherwise defined, all technical and scientific terms used in the present invention have the same meaning as commonly understood by a person of ordinary skill in the art. In addition to the specific methods, apparatuses and materials used in the examples, the present invention may also be implemented by using any method, apparatus and material in the prior art that are similar to or equivalent to the methods, apparatuses and materials described in the examples of the present invention according to the prior art mastered by a person skilled in the art and the records of the present invention.

In the following examples, a series of molecular constructs carrying a target gene were constructed based on codon optimization, ITR sequences, UTR and WPRE, and promoters, and cloned into the recombinant adeno-associated virus. In 293T cells, the corresponding adeno-associated virus was packaged to infect a differentiated muscle cell line with a certain biological titer of virus. Quantitative detection and comparison were carried out for FIX generated in cell supernatants. An AAV vector with better performance in the cells was selected and injected into hind leg muscles of C57 mouse for in-vivo comparison and screening.

The main constructs were shown in Table 1 below:

| Vector No. | Vector structure |
|---|---|
| HA21-01 | 130-CAG-kozak-F9IDTM-SV40 |
| HA21-02 | 141-CAG- kozak-F9IDTM-SV40 |
| HA21-03 | 145-CAG- kozak-F9IDTM-SV40 |
| HA21-04 | 141-CAG-UTR- kozak-F9IDTM-WPRE-SV40 |
| HA21-05 | 141-CMV- UTR- kozak F9IDTM-WPRE-SV40 |
| HA21-06 | 141-CBH- UTR- kozak F9IDTM-WPRE-SV40 |
| HA21-07 | 141-CAGG UTR- kozak -UTR-F9IDTM-WPRE-SV40 |
| HA21-08 | 141-tMCK- UTR- kozak F9IDTM-WPRE-SV40 |

### Example 1: Design of coagulation factor IX gene expressing AAV vector

An expression cassette was designed based on an amino acid sequence of a human coagulation factor IX on Uniprot{UniProtKB-P00740 (FA9_HUMAN)}, wherein this sequence contained a signal peptide of 46 amino acids and a FIX sequence of 415 amino acids. The 338^{th} amino acid of mature FIX was mutated from arginine R to leucine L. This mutation could increase the titer of FIX by more than 5 times on mouse. After the amino acid sequence was determined, IDT had undergone codon optimization on line to obtain a F9IDTM nucleotide sequence. (see SEQ ID NO: 23 for the nucleotide sequence that had undergo codon optimization, and SEQ ID NO: 24 for the amino acid sequence)

This F9IDTM gene expression cassette was designed to be inserted into an adeno-associated virus vector backbone pAAV-MCS-TTR-130-CAG-SV40. This AAV vector contained a CAG promoter, a polyadenylation signal of a SV40 virus (SV40SL), a reverse terminal repeat (ITR) and other elements. In this vector, the upstream ITR is 130 bp, and the downstream ITR is 141 bp, named as HA21-01.

On the basis of the adeno-associated virus vector HA21-01, two other vectors, HA21-02 and HA21-03, were designed, respectively. The upstream and downstream ITRs of HA21-02 were both 141 bp, and the upstream and downstream ITRs of HA21-03 were both 145 bp.

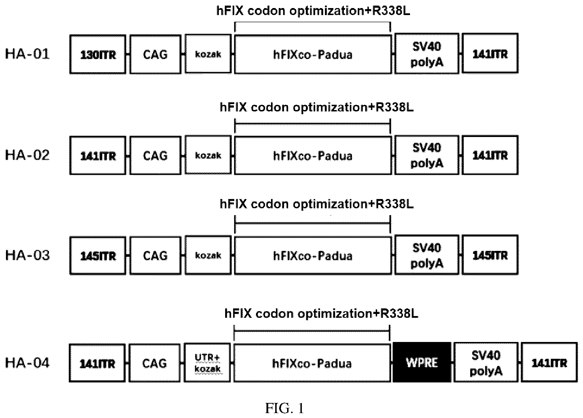

Based on the adeno-associated virus vector HA21-02, an adeno-associated virus vector HA21-04 was designed, a UTR sequence was added behind a promoter, and a WPRE sequence was added between a target gene and SV40. The structures of various adeno-associated virus vectors were shown in FIG. 1.

### Example 2: Construction of coagulation factor IX gene expressing adeno-associated virus vector

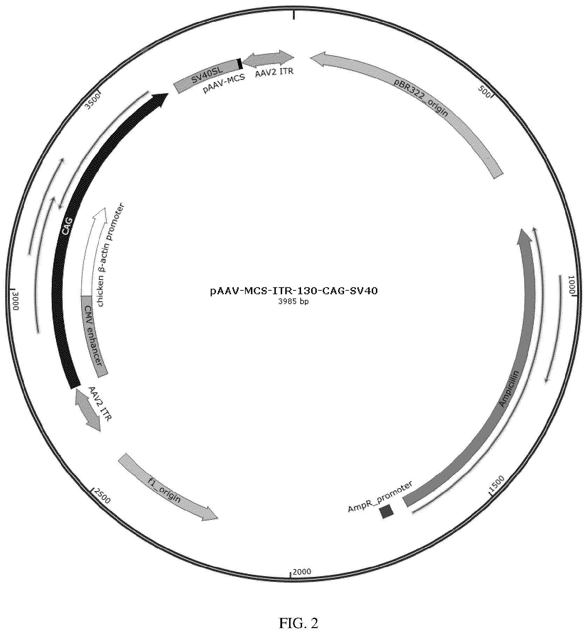

A coagulation factor F9IDTM gene expression cassette designed in Example 1 was synthesized by Nanjing GenScript and cloned between AgeI/SalI of a plurality of cloning sites in an adeno-associated virus vector backbone pAAV-MCS-ITR-130-CAG-SV40 by a well-known method of homologous recombination in the art, and named as HA21-01 (nucleotide sequence SEQ ID NO: 22). An adeno-associated virus vector construct backbone was derived from a self-provided backbone, pAAV-MCS-ITR-130-CAG-SV40 (nucleotide sequence SEQ ID NO: 1), of Kanglin Biotechnology (Hangzhou) Co., Ltd., see FIG. 2.

A 141 bp ITR sequence (nucleotide sequence SEQ ID NO: 2) was synthesized by Nanjing GenScript Biotechnology Co., Ltd., and cloned between NarI/MluI of a plurality of cloning sites on HA21-01 by a well-known method of homologous recombination in the art. The sequence information was confirmed by sequencing after cloning, named as HA21-02 (nucleotide sequence SEQ ID NO: 3).

A 145 bp ITR sequence (nucleotide sequence SEQ ID NO: 4) was synthesized by Nanjing GenScript Biotechnology Co., Ltd., and cloned between NarI/MluI and between PmeI/Pci I of a plurality of cloning sites on HA21-01 by a well-known method of homologous recombination in the art. The sequence information was confirmed by sequencing after cloning, named as HA21-03 (nucleotide sequence SEQ ID NO: 5).

A post-transcriptional regulatory element (WPRE) of a hepatitis B virus in marmot (WPRE) (nucleotide sequence SEQ ID NO: 6) was synthesized by Nanjing GenScript. A UTR sequence (nucleotide sequence SEQ ID NO: 7) was linked to a primer by the well-known PCR amplification method in the art to amplify a CAG promoter, and meanwhile F9IDTM was amplified and then cloned between upstream ITR of HA21-02 and SV40 pA signal by the well-known homologous recombination method. After cloning, the sequence information was confirmed by sequencing and named HA21-04 (nucleotide sequence SEQ ID NO: 8).

### Example 3: Virus packaging of coagulation factor IX gene expressing adeno-associated vector

Coagulation factor FIX gene adeno-associated virus expression vectors (HA21-01, HA21-02, HA21-03, HA21-04) constructed in Example 2, a capsid plasmid (KL-pAAV-RC6.2FF, having a nucleotide sequence shown in SEQ ID NO: 9) and a packaging plasmid (pHelper, having a nucleotide sequence shown in SEQ ID NO: 10) were co-transfected with 293T cells (purchased from the American Type Culture Collection (ATCC) and having a preservation number CRL-3216). In this 293T cell line, an adeno-associated virus vector for coagulation factor IX gene therapy was packaged.

The transfection method was PEI cationic polymer-mediated transient transfection of eukaryotic cells. A PEI cationic polymer was a PEI-Max transfection reagent purchased from Polysciences (purchased from Polysciences, Cat. No.: 24765-1). The transfection operation was performed according to standardized operations recommended by the manufacturer, and a transfection scale was a 15 cm² cell culture dish.

72 h after the completion of transfection, a recombinant adeno-associated virus (AAV) was harvested, and a supernatant and cells were collected and centrifuged at 4200 rpm for 10 min. The supernatant and cells were isolated after centrifugation. The cells were added with lysate and ribozyme, lysed and digested for 1 h, and centrifuged at 10000 g for 10 min. A lysate supernatant was taken. The lysate supernatant and a medium supernatant were mixed, purified by affinity chromatography, and then stored in aliquots and frozen at -80°C for later use.

5 µL of purified AAV was added to 45 µL of QE lysate (QuickExtract^{™} DNA Extraction Solutionr) (purchased from Lucigen, Cat. No. QE09050), and AAV was lysed in accordance with the procedures in the following Table 2 with a PCR instrument.

**Table 2 PCR procedures**

| Temperature | Time |
|---|---|
| 65°C | 15 min |
| 68°C | 15 min |
| 95°C | 10 min |

The physical titer of AAV was calculated based on quantitative PCR results and an AAV standard substance (AAV2 was purchased from ATCC) using a well-known method in the art.

Primer probe sequences used for quantitative PCR were:
ITR-For primer: GGAACCCCTAGTGATGGAGTT (SEQ ID NO: 11)
ITR probe: 5'-FAM-CACTCCCTCTCTGCGCGC-BHQ1-3' (SEQ ID NO: 12)
ITR-Rev primer: CGGCCTCAGTGAGCGA (SEQ ID NO: 13)

### Example 4: Protein expression detection of coagulation factor FIX gene expressing adeno-associated virus vector after transduction of C2C12 cells

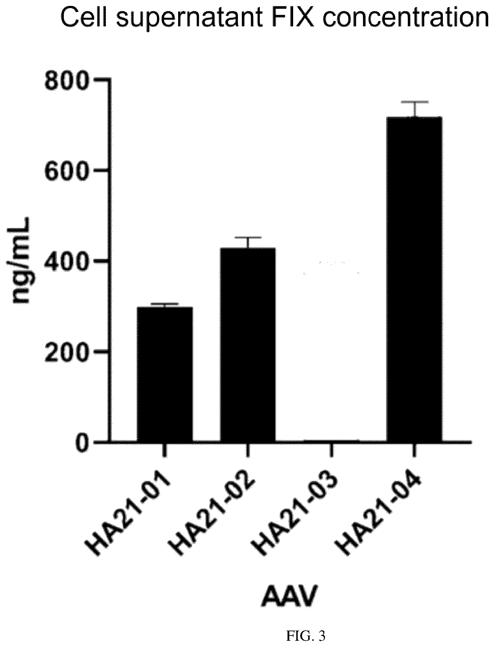

C2C12 cells were added into a 24-well cell culture plate, with 1E5 cells per well. The cells were differentiated with a 2% horse serum medium. Each coagulation factor FIX gene adeno-associated virus was packaged and purified in the above Example 3 and infected with differentiated C2C12 cells according to the MOI of 1E5. A supernatant was collected at 96 h, and detected with a human coagulation factor IX (FIXa) kit (ELISA) (purchased from Ruixinbio, Cat. No.: SU-BN16070). The assay results showed that the ITR length was 141 bp, which was obviously increased compared with 130 bp, and the expression quantity increased significantly after the addition of UTR and WPRE. The results were shown in FIG. 3.

### Example 5: Design of different promoters of coagulation factor IX gene expressing AAV vector

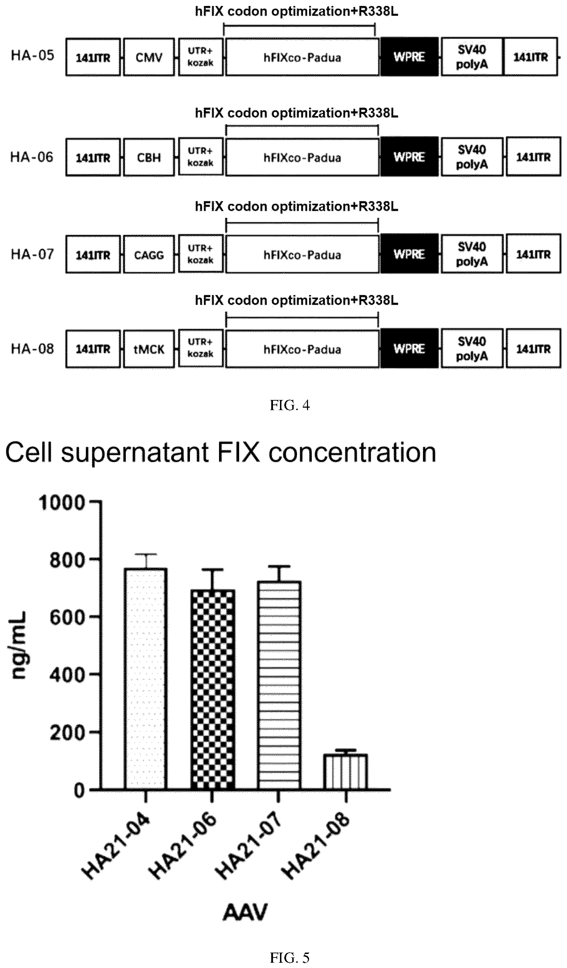

According to the preliminary study, the effect of intramuscular injection was better, so whether a muscle-specific promoter was selected for intramuscular injection was further considered. Therefore, several general promoters and muscle-specific promoters were designed for systematic comparison in this example, see FIG. 4.

### Example 6: Construction of different promoters of coagulation factor IX gene expressing adeno-associated virus vector

A CMV promoter (nucleotide sequence of SEQ ID NO: 14) was synthesized by Nanjing Genscript Biotechnology Co., Ltd., and cloned between MluI and Agel of HA21-04 by a well-known method of homologous recombination in the art to replace a CAG promoter. The sequence information was confirmed by sequencing after cloning, named as HA21-05 (nucleotide sequence of SEQ ID NO: 15).

A CBH promoter (nucleotide sequence of SEQ ID NO: 16) was synthesized by Nanjing Genscript Biotechnology Co., Ltd., and cloned between MluI and Agel of HA21-04 by a well-known method of homologous recombination in the art to replace a CAG promoter. The sequence information was confirmed by sequencing after cloning, named as HA21-06 (nucleotide sequence of SEQ ID NO: 17).

A CAGG promoter (nucleotide sequence of SEQ ID NO: 18) was synthesized by Nanjing Genscript Biotechnology Co., Ltd., and cloned between MluI and Agel of HA21-04 by a well-known method of homologous recombination in the art to replace a CAG promoter. The sequence information was confirmed by sequencing after cloning, named as HA21-07 (nucleotide sequence of SEQ ID NO: 19).

A tMCK-specific promoter (nucleotide sequence of SEQ ID NO: 20) was synthesized by Nanjing Genscript Biotechnology Co., Ltd., and cloned between MluI and Agel of HA21-04 by a well-known method of homologous recombination in the art to replace a CAG promoter. The sequence information was confirmed by sequencing after cloning, named as HA21-08 (nucleotide sequence of SEQ ID NO: 21).

### Example 7: Protein expression detection of coagulation factor FIX genes of different promoters expressing adeno-associated virus after transduction of C2C12 cells

Different promoter constructs (CAG, CBH, CAGG, tMCK) in Example 5 were subjected to AAV packaging, purification and titer assay using the method in Example 3, and the expression quantity of a cell supernatant FIX was detected by the same method. The results showed that the CAG, CBH, and CAGG promoters were significantly better than those of a tMCK promoter. The results were shown in FIG. 5.

### Example 8: Comparison of expression levels of different AAVs in animals

HA21-02 and HA21-04 adeno-associated viruses were selected and injected into 8-week-old C57 mouse at the same dose of 2.5×10¹¹VG through intramuscular injection on the lateral thighs of mouse (n=5). At different time points after injection, blood was collected from the orbit of mouse, and 3.2% sodium citrate anticoagulant (a ratio of 1: 9) was added during blood collection. After the blood was centrifuged, the FIX activity in the plasma was detected. A FIX test kit was Biophen FIX:C (Chromogenic assay for measuring Factor IX:C in plasma, or in concentrates), the Cat. No. was 221806, and a standard substance was a recombinant human coagulation factor IX for injection produced by Pfizer, 250 IU/bottle, registration certificate number: S20120053.

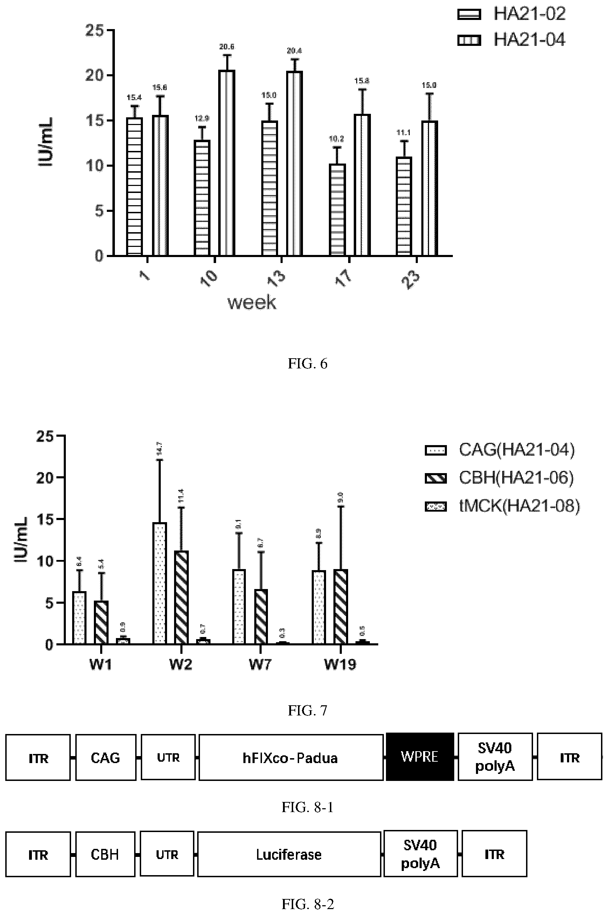

The data showed that the intramuscular administration method could play a good role. Compared with HA21-02, HA21-04 increased UTR and WPRE elements. These two elements also had a greater effect on FIX expression in vivo. As shown in FIG. 6, the specific activity values were shown in Table 3.

**Table 3 Comparison of in-vivo levels of C57BL/6J in two constructs**

| HA21-02 FIX: C IU/mL | | | | | |
|---|---|---|---|---|---|
| Time C57Bl/6J | Week 1 | Week 10 | Week 13 | Week 17 | Week 23 |
| Mouse 1 | 18.69 | 15.5 | 17.16 | 16.07 | 15.49 |
| Mouse 2 | N/A | 16.96 | 21.48 | 12.9 | 14.18 |
| Mouse 3 | 12.83 | 10.27 | 11.54 | 8.033 | 10.8 |
| Mouse 4 | 15.45 | 11.64 | 11.97 | 6.769 | 8.1 |
| Mouse 5 | 14.55 | 10.12 | 12.78 | 7.198 | 6.7 |
| Mean | 17.73 | 12.90 | 14.99 | 10.19 | 11.05 |

| HA21-04 FIX: C IU/mL | | | | | |
|---|---|---|---|---|---|
| Time C57Bl/6J | Week 1 | Week 10 | Week 13 | Week 17 | Week 23 |
| Mouse 1 | 23.46 | 24.74 | 21.74 | 25.24 | 25.36 |
| Mouse 2 | 15.46 | 21.23 | 24.58 | 18.31 | 17.75 |
| Mouse 3 | 11.08 | 15.11 | 19.01 | 11.54 | 10.4 |
| Mouse 4 | 14.13 | 22.38 | 20.26 | 12.04 | 11 |
| Mouse 5 | 13.89 | 19.57 | 16.6 | 11.69 | 10.7 |
| Mean | 15.60 | 20.61 | 20.44 | 15.76 | 15.04 |

### Example 9: Comparison of expression levels of AAVs of different promoters in animals

HA21-04, HA21-06 and H21-08 adeno-associated viruses were selected and injected into 8-week-old C57 mouse at the same dose of 2.5×10¹¹VG through intramuscular injection on the lateral thighs of mouse (n=5). Plasma at different time points was detected according to the method of Example 8.

The data showed that CAG (HA21-04) and CBH (HA21-06) had significant advantages over muscle-specific promoters in vivo, as shown in FIG. 7.

After comprehensive comparison, HA21-04 could greatly improve the expression activity of FIX, and the specific activity values were shown in Table 4.

**Table 4 Comparison of in-vivo levels of C57BL/6J in constructs of different promoter**

| HA21-04 FIX: C IU/mL | | | | | HA21-06 FIX: C IU/mL | | | | |
|---|---|---|---|---|---|---|---|---|---|
| Time C57Bl/6J | Week 1 | Week 2 | Week 7 | Week 19 | Time C57Bl/6J | Week 1 | Week 2 | Week 7 | Week 19 |
| Mouse 1 | 7.9 | 20.62 | 12.72 | 10.03 | Mouse 1 | 1.17 | 3.43 | 1.83 | 1.62 |
| Mouse 2 | 8.49 | 22.65 | 12.77 | 13.22 | Mouse 2 | 6.72 | 11.07 | 5.12 | 5.48 |
| Mouse 3 | 2.29 | 3.64 | 2.98 | 4.08 | Mouse 3 | 4.29 | 10.49 | 7.03 | 7.72 |
| Mouse 4 | 5.99 | 13.41 | 10.62 | 9.34 | Mouse 4 | 4.73 | 15.76 | 5.34 | 8.73 |
| Mouse 5 | 7.41 | 13 | 6.4 | 7.78 | Mouse 5 | 9.91 | 16.02 | 13.94 | 21.63 |
| Mean | 6.42 | 14.66 | 9.1 | 8.89 | Mean | 5.36 | 11.35 | 6.65 | 9.04 |

| HA21-08 FIX: C IU/mL | | | | | | | | | |
|---|---|---|---|---|---|---|---|---|---|
| Time C57Bl/6J | Week 1 | Week 2 | Week 7 | Week 19 | | | | | |
| Mouse 1 | 0.75 | 0.91 | 0.31 | 0.51 | | | | | |
| Mouse 2 | 0.77 | 0.61 | 0.32 | 0.56 | | | | | |
| Mouse 3 | 0.91 | 0.63 | 0.29 | 0.42 | | | | | |
| Mouse 4 | 1.09 | 0.74 | 0.34 | 0.57 | | | | | |
| Mouse 5 | 0.73 | 0.5 | 0.31 | 0.39 | | | | | |
| Mean | 0.85 | 0.68 | 0.31 | 0.49 | | | | | |

In the following examples, several potentially suitable vector types were screened first as a hemophilia gene therapy delivery system to be selected. The selected serotypes included AAV1, AAV2, AAV6, AAV6.2FF, AAV7, AAVrh74, AAV8, and AAV9. Capsid sequences of the above serotypes were synthesized commercially and cloned into an AAV packaging vector KL-pAAV-Rep through a reasonable molecular biological vector construction strategy. An AAV vector was packaged and purified to obtain several different serotypes of AAV products, including a vector (AAV-HA21) carrying a gene sequence coding a FIX-co-padua protein and a corresponding version of a vector (AAV-Luc) carrying expression regulatory elements such as a Firefly-Luciferase reporter and the same promoter.

Then, bio-distribution characteristics of different serotype vectors were explored on wild-type c57 mouse using Firefly-Luciferase reporter vectors packaged with different serotypes by using an in-vivo imaging technology, and a serotype type (AAV6.2FF) with more muscle targeting properties and less liver targeting properties was screened out.

The gene delivery efficiency of an AAV6.2FF vector was then verified in a transgenic mouse F9-KO3 from which a FIX gene was knocked out (Biomodel Organism, strain name: C57BL/6-F9em3Smoc, Cat. No.: NM-KO-200607). A tail-clip test and other proof-of-concept methods demonstrated that a gene therapy drug KL-HA21, which coded a target gene of a functionally normal FIX protein (FIX-co-padua) delivered by an AAV6.2FF system, could alleviate the symptoms of hemophilia B in F9-KO3 mouse safely and significantly for a long term through muscle-targeted gene delivery.

### Example 10: Selection, vector design and construction of different serotypes of AAVs

A subset of vector serotypes associated with muscle delivery and muscle targeting were emphatically screened by United States Clinical Trials Registry (https://clinicaltrials.gov/) and United States National Bioinformatics Center (https://www.ncbi.nlm.nih.gov), including AAV1, AAV2, AAV6, AAV6.2FF, AAV7, AAVrh74, AAV8, and AAV9. Through the database search of United States National Bioinformatics Center (https://www.ncbi.nlm.nih.gov) and Addgene, a non-profit organization that shares plasmids by scientists worldwide (http://www.addgene.org), combined with the analysis of a number of literatures related to new serotype modification strategies, nucleotide sequences of the eight serotype vectors mentioned above were sorted out. The packaging vector sequences of the corresponding serotypes were designed and constructed using a molecular biology tool SnapGene. DNA fragments were synthesized by Nanjing GenScript Biotechnology Co., Ltd., and then cloned onto an AAV packaging vector KL-pAAV-Rep by SmaI+AgeI double digestion and T4 DNA ligase ligation. After cloning, the sequence information was confirmed by sequencing. The packaging vectors of different serotypes were named as KL-pAAV-RC1, KL-pAAV-RC2, KL-pAAV-RC6, KL-pAAV-RC6.2FF, KL-pAAV-RC7, KL-pAAV-RCrh74, KL-pAAV-RC8, and KL-pAAV-RC9, having the nucleotide sequences shown in SEQ ID NO: 25-27, 9, and 28-31 in sequence.

### Example 11: Production of AAV-HA21 and AAV-Luc of different serotypes

The packaging vector plasmids of different serotypes obtained in Example 10 (KL-pAAV-RC1, KL-pAAV-RC2, KL-pAAV-RC6, KL-pAAV-RC6.2FF, KL-pAAV-RC7, KL-pAAV-RCrh74, KL-pAAV-RC8, KL-pAAV-RC9), a helper plasmid (KL-pAAV-Helper, having a nucleotide sequence of SEQ ID NO: 34), a target gene expression plasmid (a KL-pAAV-HA21 vector comprising a FIX-co-padua target gene, having a nucleotide sequence of SEQ ID NO: 32 or a KL-pAAV-Luc vector comprising a Firefly-Luciferase target gene, having a nucleotide sequence of SEQ ID NO: 33) were co-transfected with 293T cells (purchased from the American Type Culture Collection (ATCC), having a preservation number of CRL-3216). AAV was packaged by a 293T system. The transfection method was PEI cationic polymer-mediated transient transfection of eukaryotic cells. A PEI cationic polymer was a PEI-Max transfection reagent (purchased from Polysciences, Cat. No.: 24765-1). The transfection operation was performed according to standardized operations recommended by the manufacturer, and a transfection scale was a 15 cm² cell culture dish.

72 h after transfection, a cell supernatant was collected along with the cells into a 50 mL centrifuge tube. The cells were separated from the supernatant by centrifugation at 4200 rpm at room temperature for 10 min using a tabletop centrifuge. The supernatant was transferred to a new 50 mL centrifuge tube, added with MgCh and ribozyme, and mixed well for later use. The remaining cells were added with ribozyme and cell lysate, set aside at room temperature for 1 h, and then centrifuged at 10000 g at room temperature for 10 min. The supernatant was transferred to the original cell supernatant, mixed well, and finally subjected to affinity purification and concentration to obtain AAV final products of different serotypes (AAV1-CBH-Luc, AAV2-CBH-Luc, AAV6-CBH-Luc, AAV6.2FF-CBH-Luc, AAV7-CBH-Luc, AAVrh74-CBH-Luc, AAV8-CBH-Luc, AAV9-CBH-Luc, AAV6.2FF-CBH-HA21).

The physical titer of AAV was calculated using a method well known in the art based on quantitative PCR results and AAV standard substances. Primer probe sequences used for quantitative PCR were:
CMV-For primer: ACTTTCCATTGACGTCAATGGGTGG (SEQ ID NO: 35)
CMV probe: 5'- CAAGTGTATCATATGCCAAGTACGCCCCC -3' (SEQ ID NO: 36)
CMV-Rev primer: AGGTCATGTACTGGGCATAATGC (SEQ ID NO: 37)

The CMV probe had a FAM fluorophore at a 5' end and a BHQ1 fluorophore at a 3' end.

### Example 12: Tissue distribution characteristic groping test for different serotypes of AAV-Luc

According to the difference in the temporal order of vector construction, several serotype AAV-Luc vectors (AAV6.2FF-CBH-Luc, AAV8-CBH-Luc, AAV9-CBH-Luc) obtained by partial preferential packaging and purification in the above Example 2 were selected, and the bio-distribution law of this part of serotype vectors was explored first in Balb/c and C57BL/6 mouse.

Experimental protocol for BALB/c mouse: 10 µl of unilateral gastrocnemius muscles, 10 µl of tibial anterior muscles, as well as 10 µl of anterior muscles and 10 µl of posterior muscles of quadriceps were injected and administered, and the day of injection was defined as Day 0 of the test, with three 8-week-old male mouse in each group. Detailed grouping was shown in Table 5.

**Table 5 Grouping of tissue distribution characteristic groping test of different serotypes of AAV-Luc in Balb/c mouse**

| Grouping | AAV Name | Administration mode | Administration volume | Dose |
|---|---|---|---|---|
| G1 | AAV9-CBH-Luc | im | 10µL*4 | 2.0E+11vg/mouse |
| G2 | AAV6.2FF-CBH-Luc | im | 10µL*4 | 2.0E+11vg/mouse |
| G3 | AAV8-CBH-Luc | im | 10µL*4 | 2.0E+11vg/mouse |

Animals were imaged in vivo on Day 3, Day 7, Day 14, and Day 21, respectively. For in-vivo imaging, mouse were intraperitoneally injected with a 15 µg/ml substrate D-luciferin (Beyotime, Cat. No.: ST196) at a dose of 200 µl/mouse; 5 min after substrate injection, the mouse were anesthetized with isoflurane gas; and 12 min after substrate injection, a small animal in-vivo imager (PerkinElmer, IVIS Spectrum) was placed in supine and prone positions, respectively, for in-vivo imaging data collection.

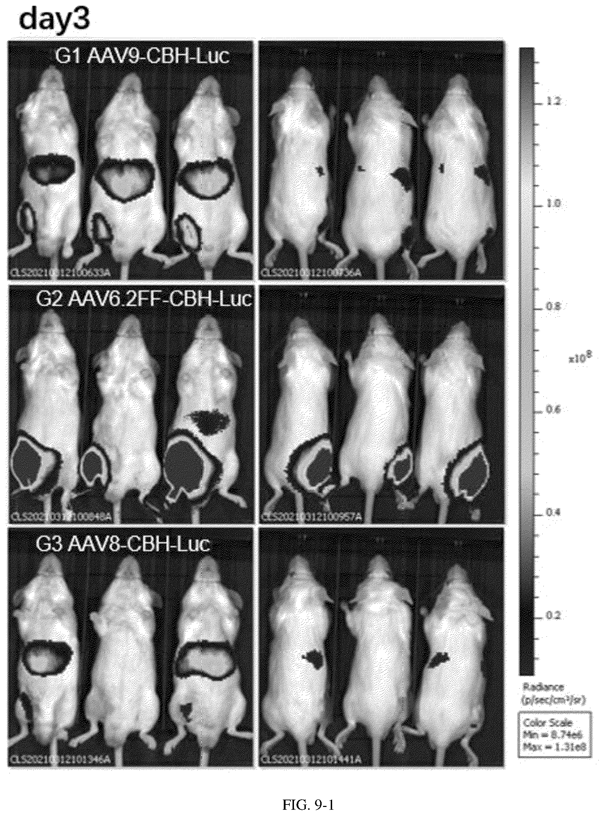

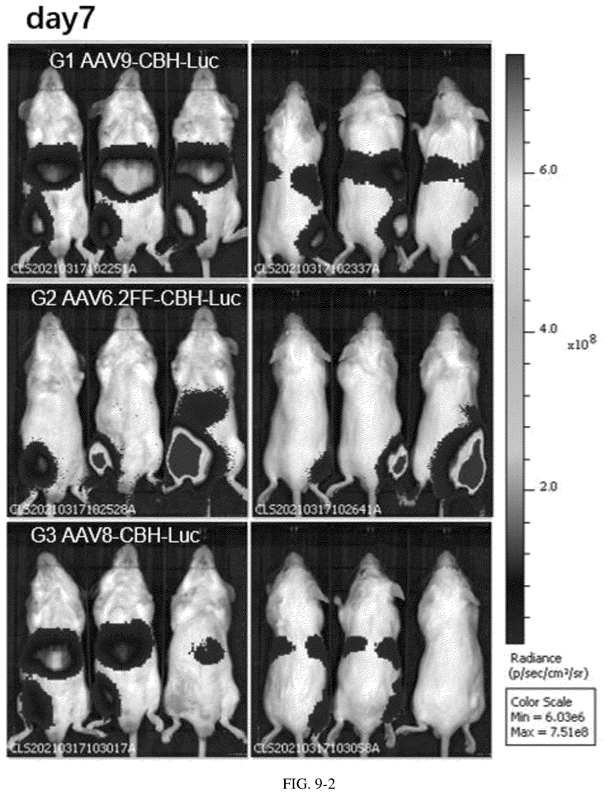

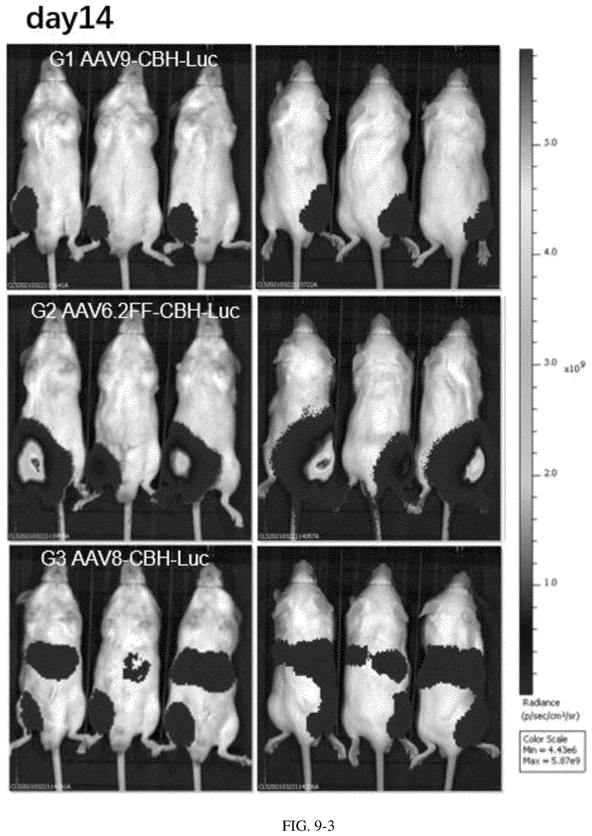

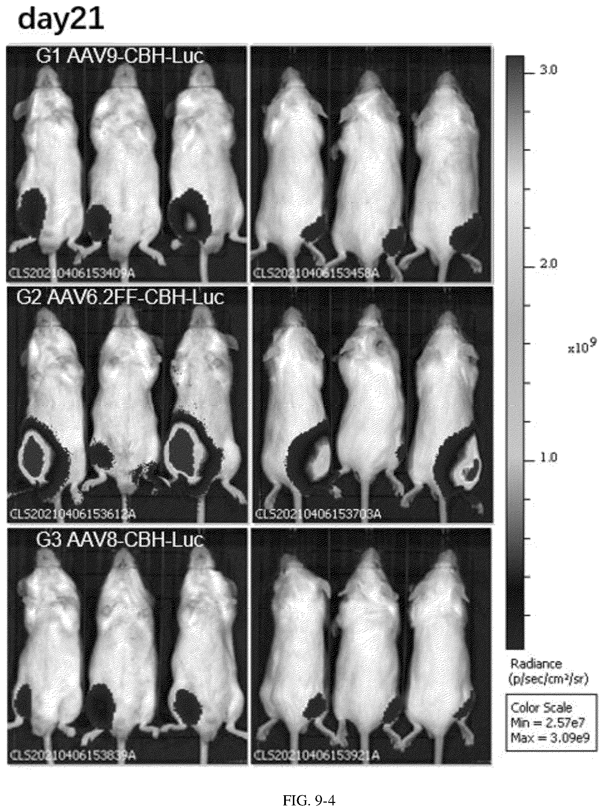

As shown in FIG. 9, each group had 3 animals, ventral and dorsal sides of each animal in each group were imaged and photographed. Observations at Day 3-Day 14 showed that AAV8-CBH-Luc and AAV9-CBH-Luc had significant liver vector expression signal distribution, while a muscle targeting property at an injection site of AAV6.2FF-Luc is very specific. There was no obvious non-specific distribution of liver or other tissues and organs.

### Example 13: Pharmacodynamic test of AAV6.2FF-HA21 in F9-KO3 model mouse

To validate the ability of AAV6.2FF to deliver a therapeutic-level FIX protein via muscle-targeted delivery, AAV was prepared from a nucleic acid construct carrying a gene sequence coding a FIX-co-padua protein (KL-pAAV-HA21, having a nucleotide sequence of SEQ ID NO. 32), as well as a packaging vector plasmid and a helper plasmid, and the AAV carrying the FIX-co-padua protein was named as AAV6.2FF-HA21 (or HA21). It was proved that AAV6.2FF-HA21 (HA21) could indeed partially or completely restore the coagulation dysfunction caused by hemophilia. The present invention selected a hemophilia B model mouse (C57BL/ 6-F9^{em3Smoc}, F9-KO3) from which a F9 gene exon and a coagulation factor IX defect was knocked out by using a gene editing technology, and verified that AAV6.2FF-HA21 could produce an effect of repairing the coagulation function of the mouse by delivering a drug in a manner of targeting muscle tissues, which is the final proof of concept of the present invention.

A total of 240 male 8-week-old F9-KO mouses were caged at a scale of ≤5 mouses/cage. The animals were free to ingest feed and drinking water for the mouse and rats. Raising conditions: corn cob padding, room temperature of 16-26°C, relative humidity of 40%-70%, and artificial lighting alternated day and night for 12/12 h.

Injection protocol: after 1 week of animal acclimatization, the mouse were injected by bilateral gastrocnemius muscles and quadricep muscles at 50 µl per point, and anesthetized with isoflurane gas at the time of injection. The day of injection was defined as Day 0 of the experiment. The mouse were randomly grouped according to their body weights. The detailed grouping was shown in Table 6.

**Table 6 Experimental grouping and dose design**

| **Grouping** | **Test substance & Reference substance (code)** | **Animal strains** | **Number of animals** | **Dose (vg/kg)** | **Administration route** | **Administration volume uL** |
|---|---|---|---|---|---|---|
| G1 | HA21-1 | F9-KO | 10*3 | 1.25E+12 | 4-point IM injection on hind legs of both sides | 50 uL*4 |
| G2 | HA21-2 | F9-KO | 10*3 | 2.50E+12 | 4-point IM injection on hind legs of both sides | 50 uL*4 |
| G3 | HA21-3 | F9-KO | 10*3 | 5.00E+12 | 4-point IM injection on hind legs of both sides | 50 uL*4 |
| G4 | HA21-4 | F9-KO | 10*3 | 1.00E+ 13 | 4-point IM injection on hind legs of both sides | 50 uL*4 |
| G5 | M | F9-KO | 10*3 | Vehicle | 4-point IM injection on hind legs of both sides | 50 uL*4 |
| G6 | P | F9-KO | 10*3 | 0.5 IU/g | IV | 200 uL |

3.2% sodium citrate 1:9 anticoagulant blood was collected at Week 1, Week 2, Week 4, Week 6 and Week 8 to detect the FIX activity and the expression quantity. After Week 4 of administration, a tail-clip test, a thromboelastogram TEG assay and APTT assay were performed, respectively.

At the same time, tissue distribution assay was set. 3 mouse were culled in each group at Week 1 and Week 4 after administration, 2 mouse were culled in each group at Week 8, and 8 mouse were raised additionally in each group. A genome was dissected, sampled and extracted according to time points, and qPCR detection was performed with a CMV primer probe. AAV DNA copies/ µ g DNA was calculated first, and then converted to AAV DNA copies/cell based on 1 ng of DNA equal to about 172 cells.

### Analysis of pharmacokinetic results of plasma concentration

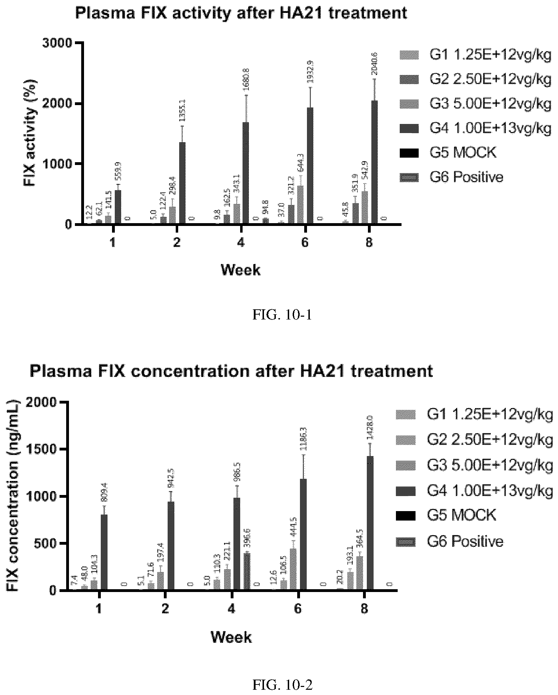

After FIX-KO mouse were injected with HA21HA21 or placebo or IV injection BeneFIX (single administration), plasma was collected from FIX-KO mouse injected with IM injection HA21 or IM injection placebo at Week 1, Week 2, Week 4, Week 6 and Week 8, respectively. The content and activity of FIX in plasma were detected (FIX Activity Assay Kit 221806, BIOPHEN; FIX Content Assay Kit SU-BN16070, Ruixinbio). The FIX-KO mouse injected with IV injection BeneFIX IV were administrated at Week 4, and plasma was collected one hour after administration. The content and activity of FIX in plasma were detected. Changes in FIX content and activity in plasma were shown in FIG. 10-1 and FIG. 10-2. Compared with a placebo group, the FIX content in each HA21 administration group and the FIX content in a BeneFIX administration group were significantly increased, and the FIX content was positively correlated with a dose of the HA21 injection group. In the HA21 administration group, the FIX activity in the plasma tended to increase with increasing concentration.

### Analysis of activated partial thromboplastin time (APTT) assay results

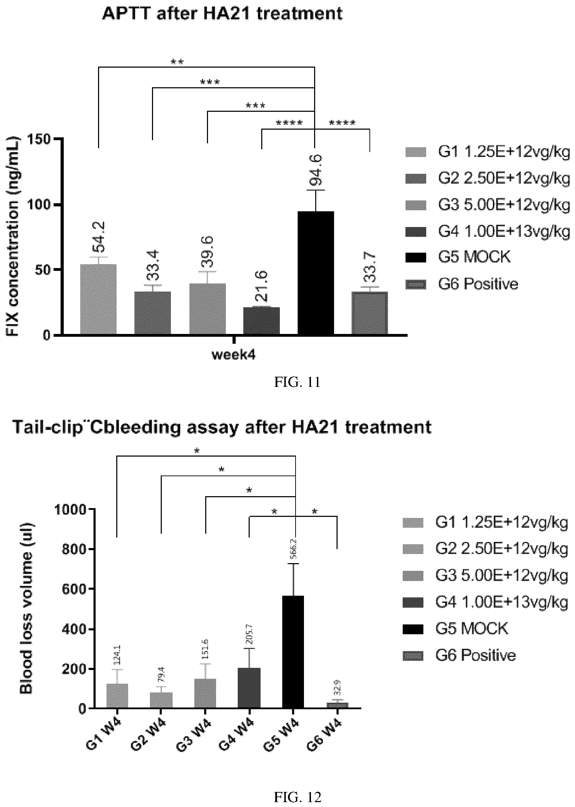

In order to detect the coagulation factor activity of an endogenous coagulation system after administration in different administration groups, APTT assay was performed at Week 8 (FIG. 11). The results showed that with the increase of HA21 administration concentrations (G1-G4), the APTT time of the tested mouse decreased, the APTT time of the HA21 administration group was significantly less than that of the placebo group, the lowest dose group had a drug effect, and the time of G3 and the time of G4 were even similar to that of a positive control group G6.

### Analysis of tail-clip test results

The tail of each mouse had a central artery, two lateral veins, and a dorsal vein, which were regularly arranged. In the tail-clip experiment, an open wound was introduced to the above blood vessels by vertical clip at a tail section area of the same size area (a diameter of 2.0 mm) of the mouse, and the wound was kept moist in a normal saline warm water bath while the outflow blood was collected. The mouse with normal coagulation ability initiate a coagulation response within a short period of time to stop further bleeding from the wound surface. Through the tail-clip test, it was evaluated that the functional improvements of the coagulation abilities of FIX-KO mouse in the administration group at each dose of HA21 versus a placebo group or a recombinant F-IX-positive control group, respectively.

As shown in FIG. 12, most of the FIX-KO mouse fail to stop bleeding from the tail section spontaneously in the placebo dose group, while the average bleeding amount was the highest among the groups. However, the average bleeding amount in the recombinant FIX positive control group was 32.92±12.72 µL, indicating that one-time exogenous FIX supplementation could significantly improve the coagulation function of mouse within a short period of time. However, the bleeding amount after tail clip was significantly reduced in the groups of all doses of HA21.

### Analsys of thromboelastogram TEG assay results

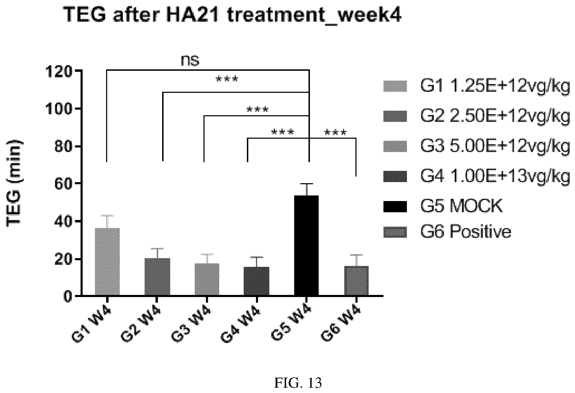

As shown in FIG. 13, in a thromboelastogram of FIX-KO mouse, except for 1 mouse in the placebo dose group, the action time of other coagulation factors exceeded an upper limit, that is, there was no coagulation function. In the recombinant FIX positive control group, the average action time of the coagulation factor was 16.34±5.84 min, indicating that exogenous FIX supplementation could significantly shorten the coagulation time. The HA21 dose group of 1.25E+12vg/kg could also shorten the coagulation time of 60% of mouse. The average coagulation time also shortened with the increase of dose, tending to the coagulation time of the recombinant FIX positive control group.

### Analysis of tissue distribution assay results

AAV DNA copy number assay showed that compared with other tissues, the copy number in gastrocnemius muscles and quadricep muscles was the highest; and at Week 1 after administration, an average copy number in gastrocnemius muscles in a highest dose group G4C was 273 copies/cell, and an average copy number in quadricep muscles was 737 copies/cell. In this experiment, the administration method was a total of four-point injections on bilateral gastrocnemius muscles and quadricep muscles, and the extremely high copy number suggested the correlation with the administration method.

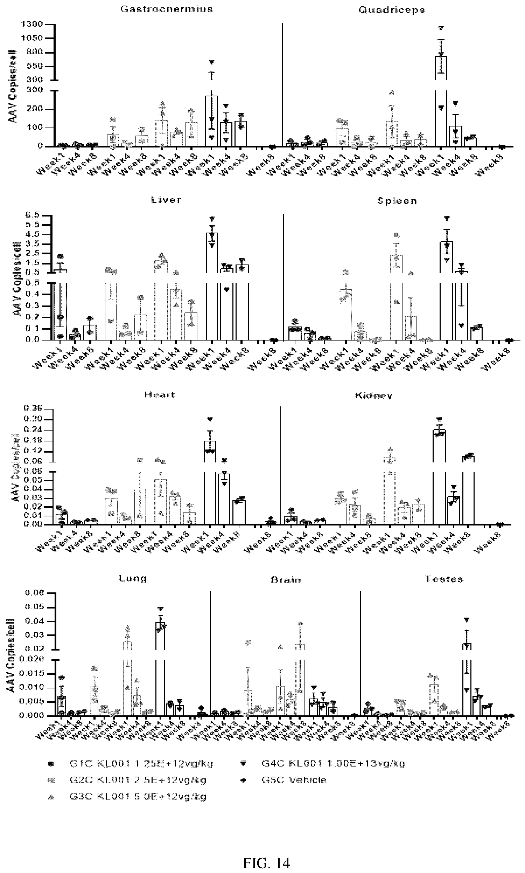

In this experiment, the copy number was higher in the liver and spleen except for muscles, but the difference with muscles was greater than 1 order of magnitude, the copy number was lower in the heart and kidney, and the copy number was extremely low in the lungs, brain, and testes. There was also a dose effect on tissue distribution. Compared with Week 1 after administration, the copy number decreased at Week 4 and Week 8 after administration. Specific data was shown in Table 7 and FIG. 14.

**Table 7 Tissue distribution data at different time points**

| **Grouping** | **Time** | **AAV copies/cell average value in various tissues at different time points** | | | | | | | | |
|---|---|---|---|---|---|---|---|---|---|---|
| | | **Gastrocnermius** | **Quadriceps** | **Liver** | **Spleen** | **Heart** | **Kidney** | **Lung** | **Brain** | **Testes** |
| G1 | Week 1 | 5.841 | 16.590 | 0.830 | 0.121 | 0.012 | 0.010 | 0.007 | 0.001 | 0.003 |
| | Week 4 | 9.117 | 26.117 | 0.053 | 0.057 | 0.003 | 0.003 | 0.001 | 0.002 | 0.001 |
| | Week 8 | 10.958 | 21.109 | 0.131 | 0.018 | 0.006 | 0.006 | 0.001 | 0.001 | 0.000 |
| G2 | Week 1 | 64.280 | 96.874 | 0.557 | 0.449 | 0.030 | 0.030 | 0.011 | 0.009 | 0.004 |
| | Week 4 | 15.038 | 22.090 | 0.080 | 0.071 | 0.009 | 0.023 | 0.003 | 0.003 | 0.001 |
| | Week 8 | 62.638 | 23.920 | 0.220 | 0.006 | 0.040 | 0.007 | 0.001 | 0.002 | 0.001 |
| G3 | Week 1 | 140.699 | 135.443 | 1.820 | 2.355 | 0.051 | 0.088 | 0.025 | 0.011 | 0.011 |
| | Week 4 | 77.859 | 34.619 | 0.449 | 0.208 | 0.032 | 0.020 | 0.007 | 0.006 | 0.004 |
| | Week 8 | 126.927 | 38.377 | 0.242 | 0.007 | 0.014 | 0.023 | 0.002 | 0.024 | 0.001 |
| G4 | Week 1 | 273.017 | 736.997 | 4.651 | 3.778 | 0.180 | 0.243 | 0.040 | 0.006 | 0.024 |
| | Week 4 | 129.345 | 111.196 | 0.963 | 0.652 | 0.058 | 0.032 | 0.004 | 0.005 | 0.007 |
| | Week 8 | 135.253 | 47.460 | 1.365 | 0.110 | 0.028 | 0.092 | 0.004 | 0.003 | 0.003 |
| G5 | Week 8 | 0.002 | 0.001 | 0.002 | 0.000 | 0.003 | 0.001 | 0.001 | 0.000 | 0.000 |

In this experiment, it was found that there were significant differences between each dose group and the placebo group by means of plasma concentration detection, APTM, tail-clip test, thromboelastogram assay and other means, which could significantly reduce the symptoms of hemophilia B in F9-KO3 mouse. The tissue distribution assay clarified the absolute advantage of muscle distribution. The effect of an AAV6.2FF-HA21 drug against hemophilia B was validated on F9-KO3 hemophilia B model mouse. The efficacy of AAV6.2FF, a serotype targeting muscle tissues, for gene therapy for hemophilia was verified.

The above examples are intended for illustrating the embodiments disclosed by the present invention and cannot be construed as the restrictions on the present invention. In addition, various modifications listed herein and the changes in the methods of the present invention are obvious to those skilled in the art without departing from the scope and spirit of the present invention. Although the present invention has been specifically described in conjunction with a variety of specific preferred examples of the present invention, it should be understood that the present invention should not be limited to these specific examples. In fact, all modifications to obtain the present invention that are obvious to a person skilled in the art, as described above, shall be included in the scope of the present invention.

## Claims

1. A nucleic acid construct, comprising the following elements:
a polynucleotide coding a coagulation factor IX; and
an AAV cis-regulatory element, comprising one or more of an ITR, a promoter, a kozak sequence, an SV40 poly A, a UTR, or a WPRE.

2. The nucleic acid construct according to claim 1, wherein a polynucleotide sequence coding the coagulation factor IX is a sequence that has undergone codon optimization; preferably, the polynucleotide sequence that has undergone codon optimization is a sequence of the coagulation factor IX comprising an R338L mutation; and preferably, the polynucleotide sequence that has undergone codon optimization is shown in SEQ ID NO: 23 or a sequence with more than 95% similarity to the sequence shown in SEQ ID NO: 23.

3. The nucleic acid construct according to claim 1, wherein the promoter is selected from a mammalian constitutive promoter, a mammalian tissue-specific promoter or a mammalian inducible promoter.

4. The nucleic acid construct according to claim 1, wherein the promoter is selected from a muscle tissue-specific promoter; and preferably, the muscle tissue-specific promoter is selected from tMCK, Desmin, SKCRM 4-DES, tMCK2, CK8, or MCK.

5. The nucleic acid construct according to claim 1, wherein the ITR has a length of 130-145 bp; preferably, the ITR has a length of 130 bp, 141 bp or 145 bp; and more preferably, the upstream ITR has the same length as that of the downstream ITR, both of which are 141 bp.

6. The nucleic acid construct according to claim 1, wherein a structure of the nucleic acid construct is selected from any of the followings:
1) ITR-promoter-kozak sequence-F9IDTM gene expression cassette-SV40 polyA sequence-ITR;
2) ITR-promoter-kozak sequence-F9IDTM gene expression cassette-WPRE-SV40 polyA sequence-ITR; and
3) ITR-promoter-UTR sequence-kozak sequence-F9IDTM gene expression cassette-WPRE-SV40 polyA sequence-ITR.

7. The nucleic acid construct according to claim 1, wherein the nucleic acid construct is an adeno-associated virus vector.

8. The nucleic acid construct according to claim 1, wherein the adeno-associated virus vector further comprises a vector backbone; and preferably, the vector backbone is pAAV-MCS-ITR-130-CAG-SV40 having a nucleotide sequence shown in SEQ ID NO: 1.

9. The nucleic acid construct according to claim 1, wherein the nucleotide sequence of the nucleic acid construct is selected from any of the followings: SEQ ID NO: 22, SEQ ID NO: 3, SEQ ID NO: 5, SEQ ID NO: 8, SEQ ID NO: 15, SEQ ID NO: 17, SEQ ID NO: 19, and SEQ ID NO: 21.

10. Use of an AAV vector serotype targeting muscle tissues in the preparation of a hereditary coagulation factor deficiency therapeutic product.

11. The use according to claim 10, wherein the AAV vector serotype targeting muscles is selected from AAV 1, AAV2, AAV6, AAV6.2FF, AAV7, AAVrh74, AAV8, AAV9 or mutants thereof.

12. The use according to claim 10, wherein the hereditary coagulation factor deficiency therapeutic product is selected from a nucleic acid construct or an adeno-associated virus.

13. The use according to claim 10, wherein the hereditary coagulation factor deficiency is selected from hemophilia A, hemophilia B or hemophilia C.

14. A nucleic acid construct for the preparation of a hereditary coagulation factor deficiency therapeutic product, wherein an adeno-associated virus prepared from the nucleic acid construct has a muscle targeting property and expresses a coagulation factor at a therapeutically effective amount; and preferably, the nucleic acid construct has a muscle targeting property under an intramuscular injection delivery mode.

15. The nucleic acid construct according to claim 14, comprising a vector backbone expressing an adeno-associated virus capsid protein; and preferably, the adeno-associated virus capsid protein vector backbone expresses an adeno-associated virus capsid protein targeting muscle tissues.

16. The nucleic acid construct according to claim 15, wherein the adeno-associated virus capsid protein vector backbone is KL-pAAV-RC1, KL-pAAV-RC2, KL-pAAV-RC6, KL-pAAV-RC6.2FF, KL-pAAV-RC7, KL-pAAV-RCrh74, KL-pAAV-RC8, or KL-pAAV-RC9, having nucleotide sequences shown in SEQ ID NOs. 25-27, 9, and 28-31 in sequence.

17. An adeno-associated virus vector expression system, comprising the nucleic acid construct according to any one of claims 1 to 9 and the nucleic acid construct according to any one of claims 14 to 16.

18. The adeno-associated virus vector system according to claim 17, further comprising a host cell.

19. The adeno-associated virus vector system according to claim 17, wherein the adeno-associated virus vector expression system is selected from an Escherichia coli expression system, a yeast expression system, an insect expression system, a mammalian expression system and a plant expression system; and preferably, the adeno-associated virus vector expression system is selected from any one of a plasmid transient transfection expression system, a baculovirus expression system, a stable transfection cell line expression system, an adenovirus expression system, and an poxvirus expression system.

20. An adeno-associated virus, wherein a adeno-associated virus is packaged by the adeno-associated virus vector system according to any one of claims 17 to 19.

21. A cell line, wherein the cell line is a cell line infected by the adeno-associated virus according to claim 20.

22. Use of the adeno-associated virus according to claim 20 and the cell line according to claim 21 in the preparation of a product for the prevention and treatment of hereditary coagulation factor deficiency.

23. The use according to claim 22, wherein the hereditary coagulation factor deficiency is hemophilia A, hemophilia B or hemophilia C.

24. A method for treating hereditary coagulation factor deficiency, comprising: administrating a patient with an effective amount of the adeno-associated virus according to claim 20.

25. The method according to claim 24, wherein the hereditary coagulation factor deficiency is hemophilia A, hemophilia B or hemophilia C.

26. The method according to claim 24, wherein the hereditary coagulation factor deficiency is mammalian hereditary coagulation factor deficiency; and preferably, the hereditary coagulation factor deficiency is human hereditary coagulation factor deficiency.

27. The method according to claim 24, wherein an effective amount of the adeno-associated virus according to claim 20 is intramuscularly injected into a patient.
